(19) Europäisches Patentamt
European Patent Office
Office européen des brevets

(11) Publication number : 0 652 215 A1

(12) # EUROPEAN PATENT APPLICATION

(21) Application number : 94307711.5

(22) Date of filing : 20.10.94

(51) Int. Cl.[6] : C07D 401/06, C07D 417/06, A01N 43/50, A01N 43/54, A01N 43/78, A01N 43/86, // (C07D401/06, 239:00, 213:00), (C07D401/06, 233:00, 213:00), (C07D417/06, 279:00, 213:00), (C07D417/06, 277:00, 213:00)

(30) Priority : 04.11.93 EP 93308824

(43) Date of publication of application : 10.05.95 Bulletin 95/19

(84) Designated Contracting States : AT BE CH DE DK ES FR GB GR IE IT LI LU NL PT SE

(71) Applicant : SHELL INTERNATIONALE RESEARCH MAATSCHAPPIJ B.V.
Carel van Bylandtlaan 30
NL-2596 HR Den Haag (NL)

(72) Inventor : Mete, Antonio
7 Bradley Drive
Sittingbourne,Kent ME10 1RB (GB)
Inventor : Mankee, Jamie Brian
66 Volante Drive
Grovehurst, Sittingbourne, Kent ME10 2JJ (GB)

(74) Representative : Allam, Peter Clerk
LLOYD WISE, TREGEAR & CO.
Norman House
105-109 Strand
London WC2R 0AE (GB)

(54) **Methylene-substituted heterocyclic compounds.**

(57) There are described novel insecticidal and acaricidal compounds of the general formula

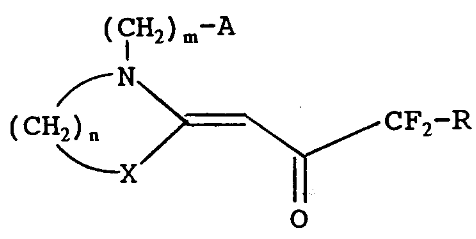

wherein X represents a methylene group, or a sulphur atom or a group of general formula $NR^1$ wherein $R^1$ represents a hydrogen atom, a $C_{1-4}$ alkyl group or a $C_{1-5}$ acyl group ;
n represents 2 or 3 ;
m represents 0, 1 or 2 ;
A represents an optionally substituted heteroaryl group ; and
R represents a chlorine, bromine or iodine atom or a $C_{1-4}$ haloalkyl group ; together with preparative processes and novel intermediates.

EP 0 652 215 A1

This invention relates to methylene-substituted heterocyclic compounds which are novel and have utility as insecticides and acaricides. The invention also relates to insecticidal and acaricidal compositions, to the use of such compounds and compositions as insecticides and acaricides, to the preparation of such compounds, and to novel intermediates, and their preparation.

The invention relates in particular to novel heterocyclic compounds having a haloalkylcarbonylmethylene substituent.

DE-A-3639877 discloses insecticidal and ectoparasitic heterocyclic compounds containing a 5- or 6- membered ring, as defined therein, the ring having at least one ring nitrogen atom, the ring being substituted by a group of general formula =Y-CO-$CF_3$ where Y represents a nitrogen atom or a group of general formula $CR^3$ where $R^3$ represents hydrogen or a group of formula -CO-$CF_3$.

It has now been found that compounds having the group -$CF_2$-X where X is a halogen substituent, but not fluorine, or is a haloalkyl group, can give rise to insecticidal and acaricidal properties which could not have been predicted, from the properties of the equivalent known compound wherein X is fluorine, or from the properties of the equivalent $CCl_3$-containing compound.

In accordance with a first aspect of the present invention there is provided a compound of the general formula

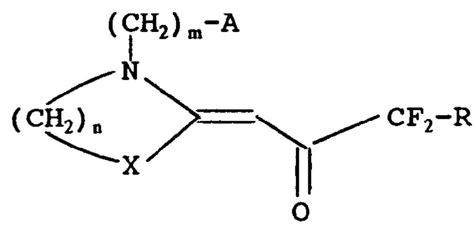

(I)

wherein X represents a methylene group, or a sulphur atom or a group of general formula $NR^1$ wherein $R^1$ represents a hydrogen atom a $C_{1-4}$ alkyl group or a $C_{1-5}$ acyl group;
n represents 2 or 3;
m represents 0, 1 or 2;
A represents an optionally substituted heteroaryl group; and
R represents a chlorine, bromine or iodine atom or a $C_{1-4}$ haloalkyl group.

Included within the scope of the present invention are standard derivatives of compounds of general formula I such as salts and oxides, for example N-oxides.

Compounds of general formula I may exist in a number of stereo-isomeric forms and tautomeric forms (e.g. keto-enol forms and cis-trans isomeric forms) and all such forms fall within the scope of the present invention.

Generally, when any of the above mentioned moieties comprises an alkyl group, the alkyl group may be linear or branched and may suitably contain 1 to 4 carbon atoms, suitable examples being methyl, ethyl and propyl. When any groups are designated as being optionally substituted, the substituent groups which are optionally present may be any of those customarily employed in the development of pesticidal compounds, and/or the modification of such compounds to influence their structure/activity, persistence, penetration or other property. In relation to moieties defined above which comprise an optionally substituted alkyl group, specific examples of such substituents include halogen, especially fluorine, chlorine, bromine or iodine atoms, and nitro, cyano, hydroxyl, $C_{1-4}$ alkoxy, $C_{1-4}$ haloalkoxy, ($C_{1-4}$ alkoxy)carbonyl groups, amino, $C_{1-4}$ alkylamino and $C_{1-4}$ alkylthio groups. It is preferred, however, that alkyl moieties are unsubstituted, or halogen-substituted. In relation to moieties defined above which comprise an optionally substituted heteroaryl group, optional substituents include halogen, especially fluorine, chlorine and bromine atoms, and nitro, cyano, amino, hydroxyl, $C_{1-4}$ alkyl, $C_{1-4}$ haloalkyl (especially $CF_3$), $C_{1-4}$ alkoxy and $C_{1-4}$ alkylthio groups. 1 to 3 substituents may suitably be employed. An acyl group is suitably an optionally substituted alkylcarbonyl group.

Suitable heteroaryl groups A may include furyl, thiophenyl, pyrazolyl, imidazolyl, pyrrolyl, 1,2,4-triazolyl, 1,2,3-triazolyl, pyrimidinyl, pyrazinyl, pyridyl, oxazolyl, isoxazolyl, 1,2,4-oxadiazolyl, 1,3,4-oxadiazolyl, thiazolyl, isothiazolyl, 1,2,5-thiadiazolyl and 1,3,4-thiadiazolyl groups. Preferred heteroaryl groups A are thiophenyl, pyrazolyl, 1,2,3-triazolyl, pyrazinyl, pyrimidinyl, pyridyl, isoxazolyl, oxazolyl, thiazolyl, 1,2,5-thiadiazolyl and 1,3,4-thiadiazolyl. A particularly preferred heteroaryl group A is a pyridyl group, especially a pyrid-3-yl group.

Suitably optional substituents for the heteroaryl group A may include fluoro, chloro, bromo, cyano, nitro, $C_{1-4}$ alkyl, $C_{1-4}$ alkoxy, $C_{1-4}$ alkylthio, $C_{1-4}$ haloalkyl, $C_{1-4}$ haloalkoxy, $C_{1-4}$ haloalkylthio, amino, mono($C_{1-4}$ alkyl)amino and di($C_{1-4}$ alkyl)amino. Preferred optional substituents are fluoro, chloro, bromo, cyano, nitro, methyl, ethyl, methoxy, methylthio, trifluoromethyl, trifluoromethoxy, trifluoromethylthio, dimethylamino or diethylamino.

A preferred optional substituent is halogen, especially chlorine.

When the group A is substituted, there may suitably be 3, 2 or, most preferably, 1 substituent(s).

An especially preferred optionally substituted heteroaryl group A is a 6-chloro-pyrid-3-yl group.

Preferably m represents 1.

Preferably X represents S or NH.

Preferably R represents a bromine or, especially, a chlorine atom or a $C_{1-4}$ fluoroalkyl, chloroalkyl or fluorochloroalkyl group, especially a $C_{1-2}$ fluoroalkyl group, preferably trifluoromethyl. Most preferably R represents a chlorine atom.

The compounds of the general formula I exhibit pesticidal, for example insecticidal or acaricidal, activity. Accordingly the invention also provides a pesticidal composition comprising a compound of the general formula I together with a carrier. The invention further provides the use of a compound or composition according to the invention, as a pesticide. The invention further provides a method of combating pests at a locus, which comprises applying to the locus a pesticidal compound or composition according to the invention.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating biocidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Compositions are often formulated and transported in a concentrated form which is subsequently diluted by the user before application. The presence of small amounts of a carrier which is a surface-active agent facilitates this process of dilution. Thus, preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing 0.5-10% w of active ingredient. Granules are usually prepared to have a size between 10 and 100 BS mesh (1.676 - 0.152 mm), and may be manufactured by agglomeration or impregnation techniques. Generally, granules will contain 0.5-75% w active ingredient and 0-10% w of additives such as stabilisers, surfactants, slow release modifiers and binding agents. The so-called "dry flowable powders" consist of relatively small granules having a relatively higher concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick "mayonnaise"-like consistency.

The composition of the invention may also contain other ingredients, for example other compounds possessing herbicidal, insecticidal or fungicidal properties.

The compounds of the present invention appear to have a broad spectrum of insecticidal and acaricidal activity, but show especially interesting activity against house flies and aphids.

In accordance with a further aspect of the present invention there is provided a process for the preparation of a compound of general formula I, comprising reacting together compounds of the general formulae II and III

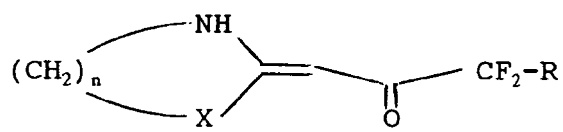 (II)

$A-(CH_2)_m-L$ (III)

where L represents a leaving moiety, and X, R, A, n and m are as defined in any of the preceding paragraphs.

Suitably the compounds of general formula II and III are reacted together under basic conditions, to effect elimination of the compound L-H. A polar aprotic solvent is preferably employed, for example dimethylformamide. The reaction may suitably be effected at a temperature in the range from 0°C to the reflux temperature. A strong base is preferably employed, suitably an alkali metal base, for example sodium hydride or potassium carbonate. Suitably the base is added first, to a compound of general formula II, at a first temperature, and after an interval the compound of general formula III is added, and the reactions suitably proceeds at a second temperature. With some compounds it is found suitable for the first temperature to be -5 to 5°C and the second temperature, 20 to 30°C. With other compounds it is found suitable for the first temperature to be 20 to 30°C and the second temperature to be the reflux temperature. Suitable leaving moieties include mesyl and tosyl groups and, especially, halogen atoms, especially chlorine.

A further aspect of the present invention relates to compounds of the general formula I, as defined above, when prepared by the process defined above.

Other processes of preparing compounds of general formula I are available, and reference is made in this regard to DE-A-3639377. This document also describes the preparation of some intermediate compounds.

Compounds of the general formula II are believed to be novel, and so constitute a further aspect of the present invention.

In accordance with another aspect of the present invention there is provided a process for the preparation of a compound of general formula II, as defined above, which comprises either of the following:

(a) reacting a compound of the general formula

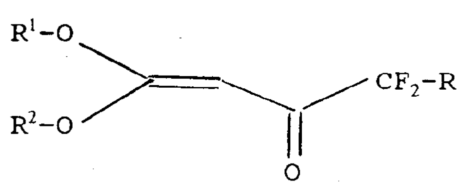 (IV)

with a compound of general formula

$NH_2-(CH_2)_n-XH$ (V)

or a salt thereof, where $R^1$ and $R^2$ independently represent alkyl groups, and R, n and X are as defined above; or

(b) reacting a compound of the general formula

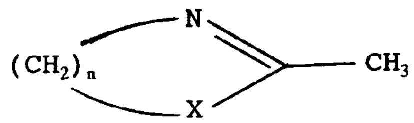

(VI)

with a compound of general formula

$$(R\text{-}CF_2\text{-}CO)_2O \qquad (VII)$$

where R, n and X are as defined above.

In relation to the reaction denoted (a) above, $R^1$ and $R^2$ are preferably both ethyl groups. An alcohol, for example ethanol, is preferably present, as a solvent. A small amount of a promotor such as DBU (1,8-diazabicyclo [5.4.0]undec-7-ene may suitably be present. The reaction may suitably be carried out at a temperature in the range 0°C to the reflux temperature.

In relation to the reaction denoted (b) above, a halogenated hydrocarbon, for example dichloromethane, is suitably present as a solvent. Alternatively or additionally an organic solvent/base, for example pyridine, may be present. The anhydride is suitably added slowly to the reaction mixture. The temperature is suitably not elevated; 0 to 40°C is preferred for most such reactions.

Compounds of general formula IV may be prepared by reacting the appropriate acid anhydride and orthoacetate compounds together, as described in Examples herein.

The invention will now be further described with reference to the following Examples.

## EXAMPLE 1

Preparation of 1-(6-chloro-pyrid-3-yl)-methyl-2-(chlorodifluoromethylcarbonylmethylene)-1,3-diazinane (R=Cl; n=3; m=1; X=NH; A=6-chloro-pyrid-3-yl)

Chlorodifluoroacetic acid anhydride (25.8 g) was added to triethyl orthoacetate (9.4 ml) and pyridine (8.1 ml) in dichloromethane (100 ml). The product, 1-(chlorodifluoromethylcarbonyl)-2,2-diethoxy ethene was a red liquid (11.2 g), which was used without further purification. Of this product, 3 g was dissolved in ethanol (25 ml), and to this was added 1,3-propanediamine (1.2 ml). The mixture was stirred overnight at ambient temperature. The solvent was removed under reduced pressure and the residue subjected to column chromatography using silica gel and dichloromethane/ethyl acetate, 50/50, v/v, as eluent. The appropriate fractions yielded 2-(chlorodifluoromethylcarbonylmethylene) -1,3-diazinane (1 g). mp 192°C.

| Analysis | | | |
|---|---|---|---|
| Calc. | 39.2% C | 4.3% H | 13.3% N |
| Found | 39.2% C | 4.0% H | 12.9% N |

This product (0.7 g) was stirred in dimethylformamide (10 ml) under a nitrogen atmosphere. Sodium hydride (0.14g of a 60% dispersion in oil) was added and stirring continued for 30 minutes, all at ambient temperature. 6-Chloro-3-(chloromethyl)pyridine (0.6 g) was added and the reaction mixture heated at 65°C for 24 hours. The mixture was then diluted with water (50 ml), and extracted twice with dichloromethane (2 x 50 ml). The organic extracts were washed with water (50 ml), dried with magnesium sulphate and evaporated to dryness under reduced pressure. The residue was subjected to column chromatography using silica gel and dichloromethane/ethyl acetate, 50/50, v/v. Appropriate fractions yielded 0.95 g of the title compound as an off-white solid. mp 148-152°C.

| Analysis | | | |
|---|---|---|---|
| Calc. | 46.4% C | 3.9% H | 12.5% N |
| Found | 45.3% C | 3.9% H | 12.2% N |

**EXAMPLE 2**

Preparation of 1-(6-chloro-pyrid-3-yl)-methyl-2-(chlorodifluoromethylcarbonylmethylene)-1,3-imidazolidine (R=Cl; n=2; m=1; X=NH; A=6-chloro-pyrid-3-yl)

2-(Chlorodifluoromethylcarbonylmethylene)-1,3-imidazolidine was prepared in accordance with the method described in Example 1 above, using ethylenediamine (0.96 g) in place of 1,3-propanediamine. The product obtained was a crystalline solid (0.5 g). mp 185°C.

| Analysis | | | |
|---|---|---|---|
| Calc. | 36.7% C | 3.6% H | 14.3% N |
| Found | 35.8% C | 3.5% H | 13.8% N |

Using this compound (1.4 g) , 6-chloro-3-(chloromethyl)pyridine (1.3 g), sodium hydride (0.3 g of a 60% dispersion in oil) and dimethylformamide (20 ml) and following the same procedure described in Example 1 above, the title compound was prepared as white needle crystals (0.35 g). mp 168°C.

| Analysis | | | |
|---|---|---|---|
| Calc. | 44.7% C | 3.4% H | 13.0% N |
| Found | 44.7% C | 3.5% H | 13.1% N |

**EXAMPLE 3**

Preparation of 3-(6-chloro-pyrid-3-yl)methyl-2-(chlorodifluoromethylcarbonylmethylene)-1,3-thiazinane (R=Cl; n=3; m=1; X=S; A=6-chloro-pyrid-3-yl)

1-(Chlorodifluoromethylcarbonyl)-2,2-diethoxyethene (4.0 g), prepared as described in Example 1 above, and 3-aminopropanethiol hydrochloride (2.2 g) were stirred in ethanol (50 ml), and 2.7 g of 1,8-diazabicyclo [5.4.0] undec-7-ene (DBU) was added. The mixture was heated at 40°C for 3 days. The ethanol was reduced under vacuum and the residue partitioned between dichloromethane (50 ml) and water (50 ml). The organic phase was washed with water (2 x 50 ml), dried and evaporated under vacuum. The residue was subjected to column chromatography using silica gel and hexane/ethyl acetate, 50/50, v/v, as eluent. Appropriate fractions yielded 1.3 g of 2-(chlorodifluoromethylcarbonylmethylene)-1,3-thiazinane.

| Analysis | | | |
|---|---|---|---|
| Calc. | 36.9% C | 3.5% H | 6.2% N |
| Found | 37.4% C | 3.7% H | 6.0% N |

Using this intermediate product (1.0 g), 6-chloro-3-(chloromethyl)pyridine (0.8 g), sodium hydride (0.2 g of a 60% dispersion in oil) and dimethylformamide (15 ml) and following the same procedure as described above in Example 1, the title compound was obtained as pale brown crystals (0.5 g). mp 163-166°C.

| Analysis | | | |
|---|---|---|---|
| Calc. | 44.2% C | 3.4% H | 7.9% N |
| Found | 43.7% C | 3.6% H | 7.7% N |

**EXAMPLE 4**

Preparation of 1-(6-chloro-pyrid-3-yl)methyl-2-(pentafluoroethylcarbonylmethylene)-1,3-diazinane ($R=CF_3$; n=3; m=1; X=NH; A=6-chloro-pyrid-3-yl)

Pentafluoropropionic anhydride (23 g) was added to triethyl orthoacetate (7.5 ml) and pyridine (6 ml) in dichloromethane (100 ml) keeping the temperature at about 25°C. The reaction mixture was stirred overnight,

then washed with water (100 ml), then with saturated sodium bicarbonate solution (100 ml), then with water (100 ml), dried and the solvent removed under vacuum to leave an orange oil (8.0 g), 1-(pentafluoroethylcarbonyl)-2,2-diethoxyethene. To 3.0 g of this intermediate product in ethanol (25 ml) was added 1,3-propanediamine (1.0 ml), and the reaction mixture was retained at ambient temperature for 18 hours. The crystalline solid which formed was filtered off and the filtrate was evaporated to give the required product, 2-(pentafluoroethylcarbonylmethylene)-1,3-diazinane (1.0 g). mp 161°C.

| Analysis | | | |
|---|---|---|---|
| Calc. | 39.3% C | 3.7% H | 11.5% N |
| Found | 40.5% C | 4.2% H | 12.3% N |

To this intermediate compound (0.9 g) in dimethylformamide (10 ml) was added 0.16 g of a 60% dispersion in oil of sodium hydride. After stirring for 30 minutes at ambient temperature, 6-chloro-3-(chloromethyl)pyridine (0.7 g) was added and the mixture heated at 65°C for 24 hours. The reaction mixture was then diluted with water (100 ml) and extracted with diethyl ether (3 x 50 ml). The ethereal extracts were combined and washed with water (50 ml), dried with anhydrous sodium sulphate and evaporated under vacuum. The residue was recrystallised from ethanol to give the title compound as orange crystals (0.5 g). mp 120°C.

| Analysis | | | |
|---|---|---|---|
| Calc. | 45.5% C | 3.5% H | 11.4% N |
| Found | 45.2% C | 3.5% H | 11.2% N |

## EXAMPLE 5

Preparation of 1-(6-chloro-pyrid-3-yl)-methyl-2-(pentafluoroethylcarbonylmethylene)-1,3-imidazolidine ($R=CF_3$; n=2; m=1; X=NH; A=6-chloro-pyrid-3-yl)

The title compound was prepared following the procedure described in Example 4 above, but using ethylenediamine (0.85 g) and 3 g of the substituted ethane. 1 g of the intermediate compound 2-(pentafluoroethylcarbonylmethylene)-1,3-imidazolidine was produced. mp 167°C.

| Analysis | | | |
|---|---|---|---|
| Calc. | 36.5% C | 3.1% H | 12.2% N |
| Found | 35.3% C | 3.2% H | 11.9% N |

Using the intermediate compound (0.5 g), 6-chloro-3-(chloromethyl)pyridine (0.4 g), 0.1 g of a 60% dispersion in oil of sodium hydride, and dimethylformamide, and the method for the final step described in Example 4 above, the title compound was obtained, as a white solid (0.3 g). mp 173°C.

| Analysis | | | |
|---|---|---|---|
| Calc. | 43.9% C | 3.1% H | 11.8% N |
| Found | 44.8% C | 3.5% H | 11.5% N |

## EXAMPLE 6

Preparation of 1-(6-chloro-pyrid-3-yl)-methyl-2-(pentafluoroethylcarbonyl-methylene)-1,3-thiazinane ($R=CF_3$; n=3; m=1; X=S; A=6-chloro-pyrid-3-yl)

3-Aminopropanethiol hydrochloride (2.5 g), 1-(pentafluoroethylcarbonyl)-2,2-diethoxy ethanol (5.0 g) and DBU (3.0 g) were refluxed in ethanol (50 ml) for 24 hours. After removal of the solvent under vacuum the residue was partitioned between dichloromethane (50 ml) and water (50 ml). The organic phase was washed twice with water (2 x 50 ml), dried, and the solvent removed under vacuum. Column chromatography using silica gel

and hexane/ethyl acetate, 50/50, v/v, as eluent yielded 2-(pentafluoroethylcarbonylmethylene)-1,3-thiazinane as a white solid (1.6 g). mp 115°C.

| Analysis | | | |
|---|---|---|---|
| Calc. | 36.8% C | 3.1% H | 5.4% H |
| Found | 37.3% C | 3.6% H | 5.5% N |

Using this intermediate product, 6-chloro-3-(chloromethyl)pyridine (0.7 g), 0.17 g of a 60% dispersion in oil of sodium hydride, and dimethylformamide (10 ml), and following the process described in Example 4 above, the title compound was prepared, as a brown crystalline solid (0.8 g). mp 156°C.

| Analysis | | | |
|---|---|---|---|
| Calc. | 43.5% C | 3.1% H | 7.2% N |
| Found | 43.8% C | 3.2% H | 7.1% N |

## EXAMPLE 7

Preparation of 3-(6-chloro-pyrid-3-yl)methyl-2-(chlorodifluoromethylcarbonylmethylene)-1,3-thiazolidine (R=Cl; n=2; m=1; X=S; A=6-chloro-pyrid-3-yl)

2-Methyl-1,3-thiazoline (1.32 g) and pyridine (2.37 g) were dissolved in dichloromethane (50 ml) and cooled in an ice-bath. Chlorodifluoroacetic anhydride (6.3 g) was added to the above solution, dropwise, over 30 minutes. The mixture was then allowed to attain ambient temperature over 18 hours. The solvent was evaporated off under reduced pressure and the residue was suspended in water (50 ml) and treated with solid sodium bicarbonate until the solution became basic. The mixture was extracted with dichloromethane (3 x 75 ml), and the combined organic extract was dried over magnesium sulphate. The solvent was removed under reduced pressure and the residue recrystallised from ethanol to give a solid (2.4 g), 2-(chlorodifluoromethylcarbonylmethylene)-1,3-thiazolidine. mp 99°C.

| Analysis | | | |
|---|---|---|---|
| Calc. | 33.7% C | 2.8% H | 6.6% N |
| Found | 34.0% C | 2.8% H | 6.8% N |

This intermediate compound (1.15 g) was dissolved in dimethyl formamide (15 ml, dried) and cooled in an ice-bath, with stirring and under a nitrogen gas atmosphere. Sodium hydride (0.3 g of a 50% suspension in oil) was added and stirring continued for 30 minutes. 6-Chloro-3-(chloromethyl)pyridine (0.89 g) was added and the mixture was stirred for 18 hours at ambient temperature. Water (50 ml) was added and the mixture was extracted with dichloromethane (3 x 75 ml). The combined organic extracts were dried over magnesium sulphate and evaporated to dryness. The residue was recrystallised from ethanol, to yield the title compound. mp 111°C.

| Analysis | | | |
|---|---|---|---|
| Calc. | 42.5% C | 3.0% H | 8.3% N |
| Found | 42.4% C | 3.1% H | 8.4% N |

## EXAMPLE 8

Preparation of 3-(6-chloro-pyrid-3-yl)methyl-2-(pentafluoroethylcarbonylmethylene)-1,3-thiazolidine ($R=CF_3$; n=2; m=1; X=S; A=6-chloro-pyrid-3-yl)

Starting with 2-methyl-1,3-thiazoline (2.0 g) and pentafluoropropionic anhydride (12.4 g) and using the procedure described in Example 7, the required intermediate product was obtained as a solid (2.2 g). mp 109°C.

| Analysis | | | |
|---|---|---|---|
| Calc. | 34.0% C | 2.5% H | 5.7% N |
| Found | 34.2% C | 2.5% H | 5.8% N |

Using this intermediate product (1.24 g) and 6-chloro-3-(chloromethyl)pyridine (0.89 g) and using the procedure described in Example 7 above, the title compound was obtained as a solid by crystallisation from ethanol (0.47 g). mp 122°C.

| Analysis | | | |
|---|---|---|---|
| Calc. | 41.9% C | 2.7% H | 7.5% N |
| Found | 41.8% C | 3.1% H | 7.8% N |

## EXAMPLE 9

Preparation of 3-(6-chloro-pyrid-3-yl)methyl-2-(n-heptafluoropropylcarbonylmethylene)-1,3-thiazolidine ($R=C_2F_5$; n=2; m=1; X=S; A=6-chloro-pyrid-3-yl)

Starting with 2-methyl-1,3-thiazoline (2.0 g) and heptafluorobutyric anhydride (16.5 g) and using the procedure described in Example 7, the required intermediate product was obtained as a solid which crystallised from ethanol (5.83 g). mp 93°C.

| Analysis | | | |
|---|---|---|---|
| Calc. | 32.3% C | 2.0% H | 4.7% N |
| Found | 32.3% C | 2.2% H | 5.2% N |

Using this intermediate compound (1.65 g) and reacting it in accordance with the procedure in Example 7, the title compound was obtained as a solid by crystallisation from ethanol (0.75 g). mp 60°C.

| Analysis | | | |
|---|---|---|---|
| Calc. | 39.8% C | 2.4% H | 6.6% N |
| Found | 39.9% C | 2.7% H | 6.7% N |

## COMPARATIVE EXAMPLE C1

Preparation of 3-(6-chloro-pyrid-3-yl)methyl-2-(trichloromethylcarbonylmethylene)-1,3-thiazolidine

2-Methyl-1,3-thiazolidine (10.12 g) and trichloroacetic anhydride (61.76 g) were reacted using the procedure described in Example 7. The required product was obtained by crystallisation from ethanol (17.92 g). mp 201°C.

| Analysis | | | |
|---|---|---|---|
| Calc. | 29.2% C | 2.5% H | 5.7% N |
| Found | 29.2% C | 2.6% H | 5.8% N |

Using this intermediate product (2.5 g) and the procedure described in Example 7, the required product was obtained by crystallisation from ethyl acetate (1.46 g). mp 125°C.

| Analysis | | | |
|---|---|---|---|
| Calc. | 38.7% C | 2.7% H | 7.5% N |
| Found | 38.7% C | 2.9% H | 7.7% N |

## COMPARATIVE EXAMPLE C2

Preparation of 3-(6-chloro-pyrid-3-yl)methyl-2-(trifluoromethylcarbonylmethylene)1,3-thiazolidine

This compound which is of the type disclosed in DE-A-3639877, was prepared in accordance with the method described therein.

## COMPARATIVE EXAMPLE C3

Preparation of 3-(6-chloro-pyrid-3-yl)methyl-2-(trifluoromethylcarbonylmethylene)1,3-thiazinane

This compound which is of the type disclosed in DE-A-3639877, was prepared in accordance with the method described therein.

## EXAMPLE 10

Pesticidal Activity

Pesticidal activity of compounds of the invention was assessed against various of the following pests:-

Spodoptera littoralis (Egyptian cotton leafworm)
Aedes aegypti (yellow fever mosquito)
Musca domestica (housefly)
Acyrthosiphon pisum (pea aphid)
Megoura viciae (vetch aphid)
Phaedon cochleariae (mustard beetle)
Trialeurodes vaporariorum (greenhouse whitefly)
Nephotettix cincticeps (green leaf hopper)
Nilaparvata lugens (brown rice plant hopper)

The test methods employed for each species appear below. In each test, unless otherwise stated, solutions or suspensions of test compound were made up over a range of concentrations in water (initially O.1%w) containing 10%w acetone and 0.025%w "TRITON X-100" (trade mark) surface active agent (the condensation product of ethylene oxide with an alkyl phenol). These solutions were sprayed at a rate equivalent to 340 litres per hectare ($3.4 \times 10^{-5} m^3/m^2$) onto Petri dishes containing either test species per se or diet onto which test species were subsequently introduced, as indicated. In some assays leaf discs infested with test species were sprayed whilst other assays involved the spraying of plants which were infested subsequently with test species after the spray solution had dried. The tests were all conducted under normal insectary conditions (23°C ± 2°C, fluctuating humidity and light). Mortality assessments were made as indicated below, in terms of percentage mortality figures. In each test a $LC_{50}$ (the dosage of active material required to kill half of the test species) for the compound was calculated from the mortality figures and compared with the corresponding $LC_{50}$ for a standard insecticide, ethyl parathion, in the same test. The results are expressed as toxicity indices thus:

$$\text{toxicity index } \frac{LC_{50}\text{ (parathion)}}{LC_{50}\text{ (test compound)}} \times 100$$

(i) Spodoptera littoralis (Diet)

Test solutions were sprayed as indicated above onto Petri dishes containing a nutritious diet for Egyptian cotton leafworm larvae. When the spray deposit had dried, each dish was infested with ten 2nd instar larvae. Mortality assessments were made 1 day (Sl-D-1d) and 7 days (Sl-D-7d) after spraying.

(ii) Spodoptera littoralis (foliar)(Sl Fol)

Test solutions were sprayed as described above onto Petri dishes containing 9cm discs of Chinese cabbage leaves on filter papers. After drying, each dish was infested with ten 2nd instar larvae. Mortality assessments were made 24 hours after infestation.

(iii) Spodoptera littoralis (ovicidal)(Sl OA)

Test solutions were sprayed as described above onto Petri dishes containing filter papers on which were approximately 50 24 hour old eggs. After 6 days the numbers of hatched and unhatched eggs were counted and percentage mortality calculated.

(iv) Aedes aegypti (Aa)

Early 4th instar larvae were used. Test solutions were made up to 0.5 ppm of test compound (and progressive half-dilutions) in water containing 0.04%w "TRITON X-100" (trade mark); acetone was initially present to aid solution, but was allowed to evaporate off before introduction of larvae.

Ten early 4th instar larvae were placed in 100 ml of test solution held at 28°C, and after 48 hours, larval mortality was recorded (Aa-2d). The final mortality was assessed by counting the number of emerged adult mosquitoes after one week (Aa-7d).

(v) Musca domestica (Md)

Batches of ten 2 to 3 day old milk-fed adult female houseflies, anaesthetised using carbon dioxide, were placed on filter papers inside Petri dishes. The dishes were sprayed with the test solutions as described above. The flies were retained in the Petri dishes and were fed with a dilute milk solution which was dripped down the side of the Petri dish and absorbed by the filter paper. Mortality was assessed after 24 hours.

(vi) Acyrthosiphon pisum (Ap)

Tests were carried out on young adult pea aphids. Whole pea plants 6 days after germination were placed on filter papers in Petri dishes. Ten aphids were transferred to each pea plant and left for 30 minutes to allow the aphids to settle and start to feed. The dishes were then sprayed with the test solutions as described above and lids were placed on the Petri dishes. Mortality was assessed after 24 hours.

(vii) Megoura viciae (Mv)

Tests were carried out on adult-Vetch aphids. Pairs of broad bean leaves on filter paper in Petri dishes were sprayed side by side with uncounted quantities of aphids in small gauze-covered containers. After passing through the spray ten aphids were tipped onto the leaves and lids were placed on the Petri dishes. Mortality was assessed after 24 hours.

(viii) Phaedon cochleariae (Pc)

Test solutions were sprayed as described above onto Petri dishes containing 9cm discs of Chinese cabbage leaves on filter papers. After drying, each dish was infested with ten adult mustard beetles (up to 1 week old). Mortality assessments were made 24 hours after infestation.

(ix) Trialeurodes vaporariorum (Tv)

French bean plants (Phaseolus vulgaris) with two fully expanded leaves were placed in a breeding culture of T. vaporariorum, also on French bean plants, which were then disturbed to ensure resettlement on the introduced plants. During the subsequent 24 hour period, eggs were deposited and kept at 27°C, with 14 hours photoperiod. All adult whiteflies were then carefully removed, leaving egg samples of a known age. After eight days the majority of eggs had hatched. Leaf discs containing the newly hatched nymphs were then cut from the leaves and transferred to moist filter paper. The discs were examined under a low-powered microscope to determine the exact number of 1st instar nymphs per disc and to remove any unhatched eggs. On average, 70-100 nymphs were found per disc.

The discs were transferred into Petri dishes and sprayed with test solutions as described above. After 6 days percentage mortalities were assessed.

(x) Nephotettix cincticeps (Nc)

Tests were carried out on young adult female green leaf hoppers. Plant pots, each containing five rice seedlings 10 to 15 cm tall arranged across the centre of the pot, were sprayed with test solutions as described above (but initial test concentration 0.05% of test compound). Spraying was on both sides of the plants with the pots horizontal. One hour after spraying, each pot was filled to the brim with fine silver sand, an open-ended glass jar was placed over each pot and each pot was infested with ten hoppers. A paper tissue was placed over the open end of each glass jar to retain the hoppers. The pots were irrigated from underneath, maintained at a temperature of 27°C ± 2°C and subjected to white fluorescent light under a regime of 18 hours light followed by 6 hours darkness. Mortality assessments were made 48 hours after infestation.

(xi) Nilaparvata lugens (Nl)

Tests were carried out on young adult female brown rice plant hoppers in the same way as for green leaf hoppers in (x) above.

## EXAMPLE 11

### Acaricidal Activity (ovicide)(Tu OA)

Acaricidal activity of the compounds of the aforementioned Examples was assessed, employing eggs of the glasshouse red spider mite, Tetranychus urticae (T.u.), less than 24 hours old, by the following procedure.

2cm diameter leaf discs cut from the leaves of French bean plants were placed on filter paper, kept moist by a cotton wool wick dipped into water.

On the day before spraying, each leaf disc was infested with 10 female adult mites. On the day of the test, the adults were removed, leaving the eggs laid overnight on the discs. The leaf discs were then sprayed with solutions of test compound made up as in Example 10 above, at a rate equivalent to 340 litres per hectare (3.4 x $10^{-5}$ $m^3/m^2$).

Throughout the test, the eggs were held under normal insectary conditions ( 23°C ± 2°C, fluctuating humidity and 16 hours days length). After 7-10 days, the numbers of hatched nymphs and unhatched eggs were assessed and the percentage mortality calculated. The $LC_{50}$ (the dosage of active material required to kill half of the test species) for the compound was calculated from the mortality figure and compared with the corresponding $LC_{50}$ for a standard insecticide, chlorfenson, in the same test. The result is expressed as toxicity index thus:

$$\text{toxicity index} = \frac{LC_{50}\ \text{(chlorfenson)}}{LC_{50}\ \text{(test compound)}} \times 100$$

Results of the assessments described in Examples 10 and 11 are provided in Table 1. In the table, a blank square indicates that testing was not carried out. The letters B and C indicate that the first test carried out at the initial test concentration of 0.1%wt (1000ppm) gave a mortality assessment of 40-69% and 0-39%, respectively. When that initial test yielded a mortality assessment of 70-100%, a rating of A would be given. Usually further testing to yield toxticity indices as described above was carried out. Toxicity indices are stated when available.

**TABLE 1**

| Compound of Example | Sl 1D | Sl 7D | Sl Fol | Sl OA | Aa 2D | Aa 7D | Md | Ap | Mv | Pc | Tv | Nc | Tu OA |
|---|---|---|---|---|---|---|---|---|---|---|---|---|---|
| 1 | C | B | <14 | 120 | C | 3 | 3 | 160 | 3 | 39 | C | 110 | <25 |
| 2 | C | 12 | B | 140 | C | 3 | <3 | 410 | 2 | | C | 350 | C |
| 3 | C | B | B | 240 | | C | 2 | 2 | 2 | 21 | C | 41 | C |
| 4 | C | C | C | B | | C | 5 | 5 | | 17 | C | 35 | B |
| 5 | C | C | C | C | | C | C | 7 | | C | C | 77 | C |
| 6 | B | B | B | 76 | | C | 12 | <1 | | C | B | 18 | C |
| 7 | <6 | 15 | 46 | C | C | <6 | 17 | 8 | | A | C | 420 | C |
| 8 | C | 11 | 32 | 39 | | 1 | 28 | <2 | | A | | 150 | C |
| 9 | C | C | <17 | C | | <2 | <5 | 8 | | A | B | C | C |
| C1 | C | C | | C | | C | C | C | | C | C | B | <20 |
| C2 | 73 | 24 | 18 | C | C | 2 | 5 | 6 | 2.5 | | C | 1000 | C |
| C3 | C | 11 | 83 | <20 | | C | 1 | 26 | | | | 29 | C |

## Claims

1. A compound of the general formula

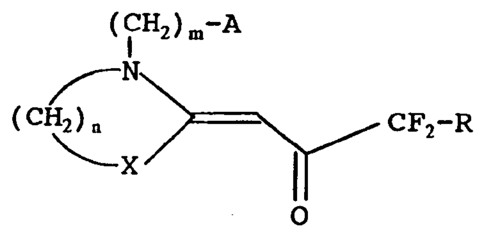

(I)

wherein X represents a methylene group, or a sulphur atom or a group of general formula $NR^1$ wherein $R^1$ represents a hydrogen atom, a $C_{1-4}$ alkyl group or a $C_{1-5}$ acyl group;
n represents 2 or 3;
m represents 0, 1 or 2;
A represents an optionally substituted heteroaryl group; and
R represents a chlorine, bromine or iodine atom or a $C_{1-4}$ haloalkyl group.

2. A compound as claimed in Claim 1, wherein A represents an optionally substituted pyridyl group.

3. A compound as claimed in Claim 1, wherein A represents a 6-chloro-pyridyl-3-yl group.

4. A compound as claimed in any preceding claim, wherein m represents 1.

5. A compound as claimed in any preceding claim, wherein X represents S or NH.

6. A compound as claimed in any preceding claim, wherein R represents a chlorine atom or a $C_{1-2}$ fluoroalkyl group.

7. A process for the preparation of a compound of general formula I, comprising reacting together compounds of the general formulae II and III

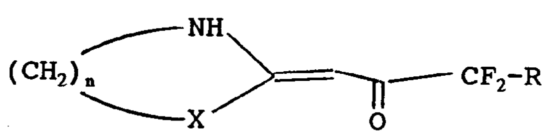

(II)

$A-(CH_2)_m-L$ (III)

where L represents a leaving moiety, and X, R, A, n and m are as described in any preceding claim.

8. A compound of the general formula I, as defined in any of Claims 1 to 6, when prepared by a process as claimed in Claim 7.

9. A compound of general formula II, per se, as defined by Claim 7.

10. A process for the preparation of a compound of general formula II, as claimed in Claim 9, which comprises either of the following:
(a) reacting a compound of the general formula

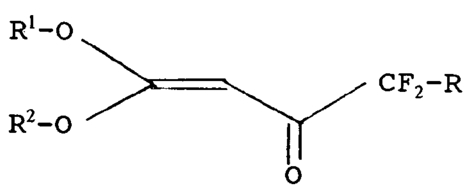

(IV)

with a compound of general formula

$NH_2-(CH_2)_n-XH$ (V)

or a salt thereof, where $R^1$ and $R^2$ independently represent alkyl groups, and R, n and X are as defined in any of Claims 1 to 6; or

(b) reacting a compound of the general formula

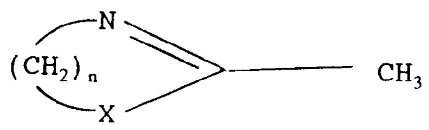

(VI)

with a compound of general formula

$$(R\text{-}CF_2\text{-}CO)_2O \qquad (VII)$$

where R, n and X are as defined in any of Claims 1 to 6.

**11.** An insecticidal or acaricidal composition, comprising a compound of the general formula I, as claimed in any of Claims 1 to 6, or Claim 8, together with at least one carrier.

**12.** A composition as claimed in Claim 11, comprising at least two carriers, at least one of which is a surface-active agent.

**13.** A method of combating insect or acaricidal pests at a locus, comprising application to the locus of a compound of the general formula I, as claimed in any of Claims 1 to 6, or Claim 8, or of a composition as claimed in Claim 11 or 12.

**14.** Use as an insecticide or acaricide of a compound of the general formula I as claimed in any of Claims 1 to 6, or Claim 8, or of a composition as claimed in Claim 11 or 12.

**15.** A compound of general formula I or II, or a process for making such a compound, or such a compound when so prepared, or an insecticidal or acaricidal method, use or composition; any such aspect being substantially as herein defined or described.

European Patent Office

# EUROPEAN SEARCH REPORT

Application Number
EP 94 30 7711

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A<br><br>D | EP-A-0 268 915 (BAYER A.G.)<br>* ex. 1,3,4,7,8,10,11 *<br>& DE-A-36 39 877<br>--- | 1-15 | C07D401/06<br>C07D417/06<br>A01N43/50<br>A01N43/54<br>A01N43/78<br>A01N43/86<br>//(C07D401/06, 239:00, 213:00), (C07D401/06, 233:00, 213:00), (C07D417/06, 279:00, 213:00), (C07D417/06, 277:00,213:00) |
| A | EP-A-0 154 178 (NIHON TOKUSHU NOYAKU SEIZO K. K.)<br>* the whole document *<br>--- | 1-15 | |
| A | EP-A-0 163 855 (NIHON TOKUSHU NOYAKU SEIZO K. K.)<br>* the whole document *<br>----- | 1-15 | |
| | | | **TECHNICAL FIELDS SEARCHED (Int.Cl.6)** |
| | | | C07D<br>A01N |

The present search report has been drawn up for all claims

| Place of search | Date of completion of the search | Examiner |
|---|---|---|
| BERLIN | 9 February 1995 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)